(19) Europäisches Patentamt / European Patent Office / Office européen des brevets

(11) **EP 3 077 060 B1**

(12) **EUROPEAN PATENT SPECIFICATION**

(45) Date of publication and mention
of the grant of the patent:
**20.03.2019 Bulletin 2019/12**

(51) Int Cl.:
*A61Q 11/00* (2006.01)     *A61K 8/22* (2006.01)
*A61K 8/23* (2006.01)

(21) Application number: **13803417.8**

(22) Date of filing: **02.12.2013**

(86) International application number:
**PCT/US2013/072557**

(87) International publication number:
**WO 2015/084296 (11.06.2015 Gazette 2015/23)**

(54) **TWO PART OXIDIZING SYSTEM FOR ORAL CARE COMPOSITIONS**

ZWEITEILIGES OXIDIERUNGSSYSTEM FÜR MUNDPFLEGEZUSAMMENSETZUNGEN

SYSTÈME OXYDANT BICOMPOSANT POUR COMPOSITIONS DE SOINS BUCCAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**12.10.2016 Bulletin 2016/41**

(73) Proprietor: **Colgate-Palmolive Company
New York, NY 10022 (US)**

(72) Inventors:
• **CHEN, Xiang
Somerset, New Jersey 08873 (US)**
• **CHOPRA, Suman K.
Monroe, New Jersey 08831 (US)**

(74) Representative: **Wibbelmann, Jobst et al
Wuesthoff & Wuesthoff
Patentanwälte PartG mbB
Schweigerstrasse 2
81541 München (DE)**

(56) References cited:
**WO-A1-03/017961     GB-A- 2 290 234**

## Description

Field

The present invention relates to a two-component oxidizing system for toothpaste compositions, that provides excellent bleaching effect together with oral care compositions containing the oxidizing system and methods and uses therefor.

Background

There is a general desire for people to have white teeth. Such white teeth are an indication of good health and in particular good oral care health. A problem is that various foods and the use of tobacco will discolor teeth. Beverages such as coffee, tea and cola beverages can discolor teeth. As a result various products and procedures have been developed to whiten teeth. These products and procedures are either purchased and/or used directly by the consumer or are applied by a dentist or other professional.

Current home treatment methods include abrasive toothpastes, toothpastes that produce oxides, whitening gels for use with a dental tray and whitening strips. The effectiveness of such techniques depends on a variety of factors including the type and intensity of the stain, the type of bleaching agent, contact time of the bleaching agent on the teeth, the amount of available bleaching active in the composition the ability of the bleaching agent to penetrate the tooth enamel, and consumer compliance. Effectiveness is also dependant on the amount of bleaching active in the composition, the ability of the active to be released during use, and the stability of the active in the product. However, the effectiveness of many of these treatments is adversely affected because of deficiencies in one or more factors relating to the composition and consumer compliance.

Thus there is a need for improved whitening (i.e. bleaching) compositions for inclusion in oral care compositions, and in particular, toothpaste compositions.

Summary

Unless otherwise indicated, the terms "%" or "percent" when used in connection with an ingredient of the toothpaste compositions of the invention is intended to refer to the percent by weight of the indicated ingredient in the toothpaste composition.

The present invention concerns an oral care composition comprising the bleaching system, wherein the ratio of peroxydisulfate to hydrogen peroxide is from 10:1 to 100:1 by weight, wherein the pH of the composition is from 7 to 8, and wherein the hydrogen peroxide is present in an amount of from 0.05% to 0.2% by weight of the oral care composition; and the peroxydisulfate is present in an amount of from 5% to 7% by weight of the oral care composition.

In some embodiments of the oral care compositions of the invention, the composition comprises one or more humectants; one or more thickeners; one or more abrasives; one or more sources of fluoride; and one or more detergents or surfactants.

In some embodiments of the oral care compositions of the invention, the composition comprises the bleaching system described herein, and: polyethylene glycol in an amount of from 0% to 23%; glycerin (99.0%-101.0%) in an amount of from 0% to 32%; propylene glycol in an amount of from 1% to 40%; polyethylene glycol/polypropylene glycol 116/66 copolymer in an amount of from 1% to 10%; polyvinyl pyrrolidone in an amount of from 0.5% to 10%; calcium pyrophosphate in an amount of from 5% to 30%; silica, for example fumed silica, in an amount of from 0% to 3%; sodium lauryl sulfate in an amount of from 1% to 3%; and tetrasodium pyrophosphate in an amount of from 0% to 3%, by weight of the composition. In some embodiments, the composition further comprises butylated hydroxytoluene in an amount of from 0.01% to 0.05% by weight of the composition. In some embodiments, the composition comprises hydrogen peroxide in an amount of 0.1% by weight of the composition, and sodium peroxydisulfate in an amount of 7% by weight of the composition.

In some embodiments of the oral care compositions of the invention, the composition further comprising one or more adjuvants selected from sweetening agents flavoring agents and coloring agents.

In some further embodiments, the invention provides a method for whitening teeth comprising contacting teeth with an oral care composition according to the invention.

In some further embodiments, the invention provides the use of a bleaching system comprising peroxide and peroxydisulfate in the manufacture of an oral care composition according to the present invention.

Detailed Description

It has been discovered in accordance with the present invention that a bleaching or oxidizing system comprising a peroxide, for example hydrogen peroxide (HP), and a peroxydisulfate, for example sodium peroxydisulfate (PDS), wherein the pH of the bleaching system is from 7 to 8, provides whitening effects that are superior to hydrogen peroxide alone.

Sodium peroxydisulfate, sometimes referred to as sodium persulfate or PDS, has the molecular formula $Na_2S_2O_8$. PDS is a strong oxidant with oxidation potential around 2.12 v, as compared to 1.77 v for HP. It has been discovered in accordance with the present invention that PDS performs best as a bleaching agent under acidic conditions, (i.e., at a pH less than 7), but nevertheless maintains significant bleaching effectiveness in neutral or slightly alkaline conditions (i.e., at pH 7-8). Although HP requires alkaline conditions for maximal effectiveness, in accordance with the present invention, PDS and low level HP (i.e., 0.01% to 1%; or from 0.01% to 0.5%; or from 0.05% to 0.3%; or from 0.05% to 0.2%; or 0.1% by weight of the oral care composition) can be formulated into an oral care composition, for example a toothpaste, at a pH near neutral (i.e., 5.8 to 7.2) which maintains the efficacy of both oxidants, and results in the composition possessing whitening power superior to 0.1% HP alone.

As used herein, "oral care composition" is intended to mean a product, which in the ordinary course of usage, is not intentionally swallowed for purposes of systemic administration of particular therapeutic agents, but is rather retained in the oral cavity for a time sufficient to contact substantially all of the dental surfaces and/or oral tissues for purposes of oral activity. The oral care composition may be in various forms including toothpaste, dentifrice, tooth gel, subgingival gel, mouthrinse, mousse, foam, mouthspray, lozenge, tooth powder, chewable tablet, chewing gum or denture product.

In one embodiment, the oral care composition is in a form selected from toothpaste, dentifrice, tooth gel, mouth rinse or denture product, with toothpaste being particularly preferred. The oral care composition may also be incorporated onto strips or films for direct application or attachment to oral surfaces.

The peroxide of the bleaching or oxidizing systems of the oral care compositions of the invention can be any of a variety of peroxides sources known to be useful in dental whitening application, for example and not limitation, hydrogen peroxide or a hydrogen peroxide source, e.g., a peroxide salt or complex (e.g., such as peroxyphosphate, peroxycarbonate, perborate, peroxysilicate, or persulphate salts; for example calcium peroxide, zinc peroxide, sodium peroxide, calcium peroxyphosphate, sodium perborate, sodium carbonate peroxide, sodium peroxyphosphate, potassium persulfate, urea peroxide, or hydrogen peroxide crosslinked polyvinyl pyrollidone complex. In some particular embodiments, the peroxide is hydrogen peroxide. Generally, hydrogen peroxide or hydrogen peroxide source is present in an amount to provide hydrogen peroxide from 0.05% to 0.2%.

The peroxydisulfate (also known as persulfate) of the bleaching or oxidizing systems of the oral care compositions of the invention can be in the forma of a metal salt, for example and not limitation the sodium, potassium, ammonium, calcium, zinc, or magnesium salt. In one preferred embodiment, the peroxydisulfate is sodium peroxydisulfate. Generally, the peroxydisulfate is present in an amount of from 5% to 7%.

The oral care compositions of the invention, for example toothpaste compositions of the invention, can include a variety of components and ingredients know in the art for toothpaste compositions. For example, the oral care compositions of the invention can include one or more humectants; one or more binders or thickeners; one or more abrasives; one or more sources of fluoride; and one or more detergents or surfactants,

Examples of suitable humectants include polyhydric alcohols (polyols) such as propylene glycol, glycerin, sorbitol, xylitol or low molecular weight polyethyleneglycols (PEGs), or polyethylene glycol/polypropylene glycol copolymers, such as for example polyethylene glycol/polypropylene glycol 116/66 copolymer. In various embodiments, humectants can prevent hardening of paste or gel compositions upon exposure to air, and also function as sweeteners. In some embodiments, the humectant system consists of one or more of polyethylene glycol in an amount of from 0% to 23%; glycerin (99.0%-101.0%) in an amount of from 0% to 32%; propylene glycol in an amount of from 1% to 40%; and polyethylene glycol/polypropylene glycol 116/66 copolymer in an amount of from 1% to 10'%. However, the presence of other humectants still providing satisfactory toothpaste properties is also contemplated.

In some embodiments, the toothpaste compositions of the invention include one or more binding and/or thickening agents. Binding agents may include polymers include polyethylene glycols, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 300,000 to about 800,000. In some embodiments, the binder or thickener system contains polyvinylpyrolidone, fumed silica, carrageenan or one or more binding agents derived from cellulose, preferably a cellulose ether, for example carboxymethylcellulose (CMC), e.g. having a medium to high degree of polymerization, e.g. 1000 to 3000, for example about 2000, e.g., in sodium salt form, e.g., CMC 2000s, in an amount effective to provide the desired viscosity and stability. Generally, the thickeners are present in the composition in an amount of up to 10%, for example from 0.1% to 10%. Examples of thickening agents include, without limitation, the binding agents described above, which also modify viscosity, for example carboxyvinyl polymers, cellulosic polymers such as hydroxyethylcellulose, carboxymethylcellulose (carmellose) and salts thereof (e.g., carmellose sodium), natural gums such as karaya, xanthan, gum arabic and tragacanth, colloidal magnesium aluminum silicate, colloidal silica, and mixtures thereof. In some preferred embodiments, the binder or thickener system comprises polyvinylpyrolidone, for example in an amount of from 0.5%) to 10%, and fumed silica, for example in an amount of up to 5% by weight of the composition.

The toothpaste compositions further comprise one or more abrasives, e.g. selected from abrasive silica and/or calcium salts, e.g. calcium carbonate and/or a calcium phosphate or pyrophosphate abrasive, e.g., tricalcium phosphate $(Ca_3(PO_4)_2)$, hydroxyapatite $(Ca_{10}(PO_4)_6(OH)_2)$, or dicalcium phosphate dihydrate $(CaHPO_4 \cdot 2H_2O$, also sometimes referred to herein as DiCal) or calcium pyrophosphate. In a particular embodiment, the abrasive includes one or both of calcium pyrophosphate in an amount of, for example from 10% to 20%, and silica, for example fumed silica, in an amount of, for example of up to 5%), for example from 1% to 3%).

The toothpaste compositions of the invention can further include one or more detergents or surfactants. Surfactants useful for the present invention include, without limitation: anionic, nonionic, and amphoteric surfactants. Surfactants may be used, for example, to provide enhanced stability of the formulation, to help in cleaning the oral cavity surfaces through detergency, and to increase foaming of the composition upon agitation, e.g., during brushing. Suitable anionic surfactants include, for example, water-soluble salts of $C_{8-20}$ alkyl sulfates, sulfonated monoglycerides of $C_{8-20}$ fatty acids, sarcosinates and taurates; for example sodium lauryl sulfate, sodium coconut monoglyceride sulfonate, sodium lauryl sarcosinate, sodium lauryl isoethionate, sodium laureth carboxylate and sodium dodecyl benzenesulfonate, and mixtures thereof. Suitable nonionic surfactants include, for example, poloxamers, polyoxyethylene sorbitan esters, fatty alcohol ethoxylates, alkylphenol ethoxylates, tertiary amine oxides, tertiary phosphine oxides, dialkyl sulfoxides, and mixtures thereof. In one embodiment, the toothpaste comprises sodium lauryl sulfate, for example in an amount of from 1% to 3%, or about 2%. The toothpaste may also or alternatively contain one or more nonpolar surfactants, for example polymers and co-polymers of ethylene glycol and propylene glycol, e.g., poloxamers, i.e., nonionic triblock copolymers composed of a central hydrophobic chain of polyoxypropylene (poly(propylene oxide)) flanked by two hydrophilic chains of polyoxyethylene (poly(ethylene oxide)). The approximate lengths of the two PEG blocks is, in some embodiments, an average of about 50-150 repeat units, e.g., about 100 repeat units while the approximate length of the propylene glycol block is an average of about 25-75 repeat unties, e.g., about 50-60 repeat units. In one embodiment, the poloxamer is poloxamer 407, also known by the BASF trade name Pluronic F127, e.g., in an amount of from 0.5% to 2%, for example about 1%. For example, in certain embodiments, the toothpaste compositions of the invention may contain both sodium lauryl sulfate and a poloxamer such as poloxamer 407. In some embodiments, the sodium lauryl sulfate is present in an amount of from 1% to 2%, for example about 2%, and the poloxamer such as poloxamer 407 is present in an amount of from 0.5% to 2%, for example about 1%, by weight of the toothpaste composition. In a preferred embodiment, the detergent or surfactant is sodium lauryl sulfate, in an amount of from 1%) to 3%) by weight of the composition.

The oral care compositions of the present invention may also contain a fluoride source - i.e., a fluoride-containing compound having a beneficial effect on the care and hygiene of the oral cavity, e.g. diminution of enamel solubility in acid and protection of the teeth against decay. Examples of suitable fluoride sources include sodium fluoride, stannous fluoride, potassium fluoride, potassium stannous fluoride

(SNFZ-KF), potassium fluorozirconate, sodium hexafluorostannate, stannous chlorfluoride, and sodium monofluorophosphate (MFP). Where present, the fluoride source would provide fluoride ion in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride. The amount by weight of these materials, which dissociate or release fluoride or fluorine-containing ions, will depend on the molecular weight of the counterion as well as on the particular application, but suitably may be present in an effective but non-toxic amount, usually within the range of 0.1 to 2% by weight. In some embodiments, a fluoride source selected from sodium fluoride, stannous fluoride, sodium monofluorophosphate and mixtures thereof, is used, for example the toothpaste of the invention may comprise an effective amount of sodium monofluorophosphate. In some embodiments, the fluoride source is sodium fluoride, which is present in an amount of from 0.15% to 0.5%, or from 0.2% to 0.3% by weight of the toothpaste composition.

The oral care compositions of the present invention may also contain one or more tartar control agents, for example pyrophosphates such as tetrasodium pyrophosphate, zinc citrate, and antibiotic compounds such as triclosan. In some embodiments, the oral care compositions of the invention comprise tetrasodium pyrophosphate, in an amount of up to 3% (i.e., from 0% to 3%) by weight of the composition.

As will be evident to one of skill in the art, some components of the invention may perform multiple functions, and the identification of a compound as having one function herein is not meant to exclude its use for other functions in a particular composition. For example, a compound such as carboxymethylcellulose or polyethylene glycol may act as a binder, but also has humectant and thickening properties.

It is also understood that compounds in formulation may naturally react, disassociate, and/or form complexes with one another. Accordingly, certain ingredients may be formed *in situ* (for example, it is understood that sodium chloride may be formed by reacting sodium hydroxide with hydrochloric acid), and also may in formulation exist in different forms (for example, to the extent the sodium chloride is dissolved, it will naturally disassociate into separate sodium and chloride ions, as opposed to a solid salt). As is usual in the art, the compositions of the invention are described in terms of exemplary formulation ingredients, without intending to exclude combinations of other ingredients that result in the same final compositions, or to exclude the natural reaction products of the described ingredient combinations.

In some embodiments described above, the toothpaste compositions of the invention can further include one or more sweetening agents, flavoring agents and coloring agents. Any suitable flavoring or sweetening material may be employed. Examples of suitable flavoring constituents include flavoring oils, e.g. oil of spearmint, peppermint, wintergreen, clove, sage, eucalyptus, marjoram, cinnamon, lemon, and orange, and methyl salicylate. Suitable sweetening agents include sucrose, lactose, maltose, xylitol, sodium cyclamate, perillartine, AMP (aspartyl phenyl alanine methyl ester), saccharine and the like. Suitably, flavor and sweetening agents may each or together comprise from about 0.1% to 5% more of the oral care composition. In some embodiments, the toothpaste compositions of the invention include one or more flavoring agents in an amount of from about 0.5% to about 3.0%; about 0.8% to about 1.6%; or about 1.2%. In some further embodiments, the toothpaste contains one or more sweetening agents in an amount of up to 0.5% by weight of the toothpaste composition; for example up to 0.3%; or from 0. 1 % to 0.2%).

Various other materials may be incorporated in the oral preparations of this invention such as whitening agents, including urea peroxide, calcium peroxide, titanium dioxide, hydrogen peroxide, complexes of polyvinylpyrolidone (PVP) and hydrogen peroxide, preservatives such as butylated hydroxytoluene, vitamins such as vitamin B6, B 12, E and K, silicones, chlorophyll compounds, potassium salts for the treatment of dental hypersensitivity such as potassium nitrate as well as antitartar agents such as sodium tripolyphosphate and di- and tetra-alkali metal pyrophosphate salts such as di- and tetrasodium pyrophosphate. These agents, when present, are incorporated in the compositions of the present invention in amounts which do not substantially adversely affect the properties and characteristics desired.

In general, each of the foregoing adjuvants may be typically incorporated in the instant toothpastes in amounts up to 5% provided they do not adversely affect the stability and cleaning properties of the non-bleeding striped dentifrice of present invention.

In some embodiments of the oral care compositions of the invention, the composition comprises the bleaching system described herein, and:

polyethylene glycol in an amount of from 0% to 23%;
glycerin (99.0%-101.0%) in an amount, of from 0% to 32%;
propylene glycol in an amount of from 1 % to 40%;
polyethylene glycol/polypropylene glycol 116/66 copolymer in an amount of from 1% to 10%;
polyvinyl pyrrolidone in an amount of from 0.5% to 10%;
calcium pyrophosphate in an amount of from 5% to 30%;
silica, for example fumed silica, in an amount of from 0.1% to 5%;
sodium lauryl sulfate in an amount of from 1% to 3%; and
tetrasodium pyrophosphate in an amount of from 0% to 3%, by weight of the composition.

The invention provides, in one embodiment, a bleaching system for oral care compositions comprising hydrogen peroxide and/or a hydrogen peroxide source and a peroxydisulfate, e.g., sodium peroxydisulfate, wherein the ratio of peroxydisulfate (by weight of peroxydisulfate without its counterion) to hydrogen peroxide is from 10:1 to 100:1 by weight, e.g., 25:1 to 75:1, e.g., 50:1 to 60:1.

(Amounts of peroxydisulfate are given by weight of peroxydisulfate without its counterion, unless otherwise indicated. Amounts of specific salts, e.g., sodium, peroxydisulfate, are given by weight of the salt, unless otherwise indicated). For example, the invention provides:

1.1. Composition 1, wherein the bleaching system comprises hydrogen peroxide and a peroxydisulfate, wherein the ratio of peroxydisulfate to hydrogen peroxide is from 10:1 to 100:1 by weight, wherein the pH of the composition is from 7 to 8, and wherein the hydrogen peroxide is present in an amount of from 0.05% to 0.2% by weight of the oral care composition; and the peroxydisulfate is present in an amount of from 5% to 7% by weight of the oral care composition.

1.2. The foregoing composition wherein the peroxide is hydrogen peroxide, and the peroxydisulfate is sodium peroxydisulfate.

1.3. The foregoing composition wherein the composition comprises:

one or more humectants;
one or more thickeners;
one or more abrasives;
one or more sources of fluoride; and
one or more detergents or surfactants.

1.4. Any foregoing composition wherein the composition comprises:

polyethylene glycol in an amount of from 0% to 23%;
glycerin (99.0%-101.0%) in an amount of from 0% to 32%;
propylene glycol in an amount of from 1% to 40%;
polyethylene glycol/polypropylene glycol 116/66 copolymer in an amount of from 1% to 10%;
polyvinyl pyrrolidone in an amount of from 1% to 10%;
calcium pyrophosphate in an amount of from 5% to 30%;
silica, for example fumed silica, in an amount of from 0.1% to 5%;
sodium lauryl sulfate in an amount of from 1% to 3%; and
tetrasodium pyrophosphate in an amount of from 0% to 3%, by weight of the composition.

1.5. Any foregoing composition further comprising butylated hydroxytoluene in an amount of form 0.01% to 0.05% by weight of the composition.

1.6. Any foregoing composition wherein the composition comprises hydrogen peroxide in an amount of 0.05% to 0.15% e.g., about 0.1% by weight of the composition.

1.7. Any foregoing composition wherein the composition comprises hydrogen peroxide in an amount of about 0.1% by weight of the composition, and peroxydisulfate in an amount of about 7% by weight of the composition.

The invention further provides, in another embodiment, a method for whitening teeth comprising contacting teeth with an oral care composition according to any of Compositions 1, et seq.

The invention further provides, in another embodiment, the use of a bleaching system comprising hydrogen peroxide and/or a hydrogen peroxide source and a peroxydisulfate, e.g., sodium peroxydisulfate, wherein the ratio of peroxydisulfate to peroxide is from 10:1 to 100:1 by weight, e.g., 25:1 to 75:1, e.g., 50:1 to 60:1 by weight; in the manufacture of an oral care composition, e.g., according to any of Compositions 1, *et seq.*

The following examples are further illustrative of the nature of the present invention, but it is understood that the invention is not limited thereto. All amounts and proportions referred to herein and in the appended claims are by weight, unless otherwise indicated.

Example 1 - Dentifrice Formulation

PDS was formulated into a commercial toothpaste having 0.1% HP. A typical composition is shown in Table 1.

Table 1

| Dentifrice Composition | |
|---|---|
| Ingredient | Percent of Composition |
| Polyethylene Glycol 600 | 8.5-22.5 |
| 99.0% - 101.0% Glycerin | 18-32 |
| Propylene Glycol USP | 15-22 |
| Polyethylene Glycol/Polypropylene Glycol 116/66 Copolymer | 7.5 |
| Hydrogen Peroxide | 0.1 |
| Sodium Peroxydisulfate | 0-14 |
| Polyvinyl Pyrrolidone | 2.5-10 |
| Sodium Saccharin | 0.6 |
| Sucralose USP | 0-0.05 |
| Calcium Pyrophosphate | 10-20 |

(continued)

| Dentifrice Composition | |
|---|---|
| Ingredient | Percent of Composition |
| Fumed Silica | 0-2.5 |
| Sodium Lauryl Sulfate | 2 |
| Butylated Hydroxytoluene | 0.03 |
| 85% Syrupy Phosphoric Acid or Sodium Hydroxide - 50% Soln. | 0-1 |
| Tetrasodium Pyrophosphate | 0-2 |
| Flavor | 1-2.5 |

Example 2 - In vitro Brushing Test

An in vitro brushing test was conducted on bovine teeth using a brushing machine. Whiteness of the bovine teeth was measured using a Spectroshade from MHT Optic Research, AG, Italy. Stained bovine teeth were prophied first to have L* value within 60-65. The dentifrice was made into 1:1 slurry using DI water. The prophied bovine teeth were mounted in molding clay in a tray which could be assembled in a brushing machine. The teeth were brushed using the dentifrice slurry for 2 minutes with 120 stroke/minute under 250 gram weight load. The whiteness of teeth, L*a*b*, after each brushing cycle was measured using the Spectroshade. The Whiteness Index, W, was calculated as

$$W* = \sqrt{a*^2 + b*^2 + (L*-100)^2}$$

, and delta W (compared to baseline W) was used to record the change of whiteness after brushing. A greater change on whiteness of teeth is reflected in a more negative value for delta W.

Example 3 - Efficacy of Dentifrice

Three dentifrices, all containing 0.1% HP, were made using the formulation in Table 1 with different loading of PDS (3.5% PDS, 7.0% PDS and 14.0% PDS). The brushing test was conducted using these dentifrices for total 14 cycles versus the control dentifrice which contains 0.1 % HP without PDS. Delta W was calculated after each brushing cycle. The results are shown listed in Table 2.

TABLE 2

| Brushing Cycle | ΔW Ave | | | |
|---|---|---|---|---|
| | Control | 3.5% PDS | 7% PDS | 14% PDS |
| Cycle 1 | -1.75 | -3.31 | -3.54 | -2.65 |
| Cycle 2 | -2.64 | -4.25 | -4.65 | -3.53 |
| Cycle 3 | -3.51 | -5.25 | -6.31 | -4.67 |
| Cycle 4 | -3.91 | -5.67 | -6.3 | -5.71 |
| Cycle 5 | -4.35 | -6.85 | -7.25 | -5.65 |
| Cycle 6 | -4.87 | -6.25 | -7.75 | -6.02 |
| Cycle 7 | -5.21 | -7.21 | -8.45 | -6.69 |
| Cycle 8 | -6.26 | -7.95 | -9.01 | -6.96 |
| Cycle 9 | -6.51 | -7.95 | -9.13 | -7.48 |
| Cycle 10 | -6.78 | -8.93 | -10.27 | -7.47 |
| Cycle 11 | -6.96 | -8.01 | -9.94 | -8.8 |
| Cycle 12 | -7.49 | -9.12 | -9.9 | -8.17 |
| Cycle 13 | -7.94 | -8.26 | -10.71 | -9.21 |

(continued)

| Brushing Cycle | ΔW Ave | | | |
|---|---|---|---|---|
| | Control | 3.5% PDS | 7% PDS | 14% PDS |
| Cycle 14 | -7.9 | -8.99 | -10.88 | -9.34 |

All three prototype dentifrices performed better than the 0.1% HP control, demonstrating that the dual bleaching system of HP and PDS improved the whitening efficacy of 0.1% HP formulation. The results also show that beyond 7.0% PDS dosing, the whitening efficacy does not enhance further, and in fact declines.

The pH values for the 1:1 dentifrice slurries used in the brushing test were determined. The results are listed below in Table 3.

Table 3 pH of Dentifrice Slurries (1:1)

| Dentifrice Slurry Description, 1:1 | pH |
|---|---|
| Control with 0.1% HP | 7.83 |
| Dentifrice #1 with 0.1% HP and 3.5% PDS | 7.56 |
| Dentifrice #1 with 0.1% HP and 7.0% PDS | 7.37 |
| Dentifrice #1 with 0.1% HP and 14.0% PDS | 7.11 |

It can be seen from the data in Table 3 that the pH of dentifrice slurry decreases with the increased PDS loading. When pH drops to 7.2~7.3, the efficacy of HP is hindered. Below that pH, conditions are not favorable for HP efficacy. However, PDS is expected to maintain its whitening efficacy near that pH range, and it performs best at lower pH conditions, i.e., around pH 6.

It is also observed that the performance of 0.1 % HP and 14.0% PDS is inferior to that of 0.1% HP and 7.0% PDS. Accordingly, it is preferred that the dual bleach system of the invention contain 7% or less PDS when the system contains 0.1 % HP.

The weight percentage of peroxydisulfate is about 80% of the weight of the sodium peroxydisulfate. For a formulation of 7.0% of PDS and 0.1% HP, therefore, the ratio of peroxydisulfate to hydrogen peroxide is about 56:1, and for a formulation of 3.5% PDS and 0.1% HP, the ratio is about 28:1

The optimum dual bleaching system contains 7.0% of PDS and 0.1% HP, therefore, the ratio of peroxydisulfate to hydrogen peroxide is about 56:1.

**Claims**

1. An oral care composition comprising a bleaching system, wherein the bleaching system comprises hydrogen peroxide and a peroxydisulfate, wherein the ratio of peroxydisulfate to hydrogen peroxide is from 10:1 to 100:1 by weight, wherein the pH of the composition is from 7 to 8, and wherein the hydrogen peroxide is present in an amount of from 0.05% to 0.2% by weight of the oral care composition; and the peroxydisulfate is present in an amount of from 5% to 7% by weight of the oral care composition.

2. The oral care composition of claim 1, wherein the composition is a toothpaste.

3. The oral care composition of claim 1 or claim 2, wherein the hydrogen peroxide is present in an amount of 0.1% by weight of the oral care composition; and wherein the peroxydisulfate is present in an amount of 7% by weight of the oral care composition.

4. The oral care composition of any preceding claim, wherein the peroxydisulfate is sodium peroxydisulfate.

5. The oral care composition of any preceding claim, wherein the composition comprises:

   one or more humectants;
   one or more thickeners;
   one or more abrasives;

one or more sources of fluoride; and
one or more detergents or surfactants.

6. The oral care composition of any preceding claim, wherein the composition comprises:

polyethylene glycol in an amount of from 0% to 23%;
glycerin (99.0%-101.0%) in an amount of from 0% to 32%;
propylene glycol in an amount of from 1% to 40%;
polyethylene glycol/polypropylene glycol 116/66 copolymer in an amount of from 1%to 10%;
polyvinyl pyrrolidone in an amount of from 0.5% to 10%;
calcium pyrophosphate in an amount of from 5% to 30%;
silica, for example fumed silica, in an amount of from 0.1% to 5%;
sodium lauryl sulfate in an amount of from 1% to 3%; and
tetrasodium pyrophosphate in an amount of from 0% to 3%, by weight of the composition.

7. The oral care composition of claim 6, further comprising butylated hydroxytoluene in an amount of from 0.01% to 0.05% by weight of the composition.

8. The oral care composition of any preceding claim, wherein the composition comprises hydrogen peroxide in an amount of 0.05% to 0.15% by weight of the composition.

9. The oral care composition of any preceding claim, further comprising one or more adjuvants selected from sweetening agents flavoring agents and coloring agents.

10. Oral care composition according to any preceding claim for use in a method for whitening teeth, said method comprising contacting teeth with the oral composition.

11. Use of a bleaching system according to claim 1 in the manufacture of an oral care composition according to any of claims 1-9.


**Patentansprüche**

1. Mundpflegezusammensetzung umfassend ein Bleichsystem, worin das Bleichsystem Wasserstoffperoxid und ein Peroxydisulfat umfasst, wobei das Verhältnis Peroxydisulfat zu Wasserstoffperoxid von 10:1 bis 100:1, bezogen auf das Gewicht, ist, wobei der pH-Wert der Zusammensetzung von 7 bis 8 ist und wobei das Wasserstoffperoxid in einer Menge von 0,05 bis 0,2 Gew.% der Mundpflegezusammensetzung vorliegt und das Peroxydisulfat in einer Menge von 5 bis 7 Gew.% der Mundpflegezusammensetzung vorliegt.

2. Mundpflegezusammensetzung nach Anspruch 1, wobei die Zusammensetzung eine Zahnpasta ist.

3. Mundpflegezusammensetzung nach Anspruch 1 oder Anspruch 2, wobei das Wasserstoffperoxid in einer Menge von 0,1 Gew.% der Mundpflegezusammensetzung vorliegt und wobei das Peroxydisulfat in einer Menge von 7 Gew.% der Mundpflegezusammensetzung vorliegt.

4. Mundpflegezusammensetzung nach irgendeinem vorhergehenden Anspruch, wobei das Peroxydisulfat Natriumperoxydisulfat ist.

5. Mundpflegezusammensetzung nach irgendeinem vorhergehenden Anspruch, wobei die Zusammensetzung umfasst:

ein oder mehrere Befeuchtungsmittel;
ein oder mehrere Verdickungsmittel;
ein oder mehrere Abrasivstoffe;
ein oder mehrere Quellen von Fluorid; und
ein oder mehrere Detergentien oder Tenside.

6. Mundpflegezusammensetzung nach irgendeinem vorhergehenden Anspruch, wobei die Zusammensetzung umfasst:

Polyethylenglucol in einer Menge von 0% bis 23%;
Glycerin (99,0% - 101,0%) in einer Menge von 0% bis 32%;
Propylenglucol in einer Menge von 1% bis 40%;
Polyethylenglucol/Polypropylenglucol 116/66-Copolymer in einer Menge von 1% bis 10%;
Polyvinyl Pyrrolidon in einer Menge von 0,5% bis 10%;
Kalziumpyrrophosphat in einer Menge von 5% bis 30%;
Silica, beispielsweise rauchende Silica, in einer Menge von 0,1% bis 5%;
Natriumlaurylsulfat in einer Menge von 1% bis 3%; und
Tetranatriumpyrrophosphat in einer Menge von 0% bis 3%, bezogen auf das Gewicht der Zusammensetzung.

7. Mundpflegezusammensetzung nach Anspruch 6, weiterhin umfassend butyliertes Hydroxytoluen in einer Menge von 0,01% bis 0,05%, bezogen auf das Gewicht der Zusammensetzung.

8. Mundpflegezusammensetzung nach irgendeinem vorhergehenden Anspruch, wobei die Zusammensetzung Wasserstoffperoxid in einer Menge von 0,05% bis 0,15%, bezogen auf das Gewicht der Zusammensetzung, umfasst.

9. Mundpflegezusammensetzung nach irgendeinem vorhergehenden Anspruch, weiterhin umfassend Hilfsstoffe ausgewählt aus Lösungsmittel, Geschmacksmittel und Farbmittel.

10. Mundpflegezusammensetzung nach irgendeinem vorhergehenden Anspruch zur Verwendung in einem Verfahren zum Weißen von Zähnen, wobei das Verfahren das in Kontakt bringen der Zähne mit der oralen Zusammensetzung umfasst.

11. Verwendung eines Bleichsystems nach Anspruch 1 bei der Herstellung einer Mundpflegezusammensetzung gemäß irgendeinem der Ansprüche 1 - 9.


**Revendications**

1. Composition de soin buccal comprenant un système de blanchiment, dans laquelle le système de blanchiment comprend du peroxyde d'hydrogène et un peroxydisulfate, dans laquelle le rapport du peroxydisulfate au peroxyde d'hydrogène est de 10:1 à 100:1 en poids, dans laquelle le pH de la composition est de 7 à 8, et dans laquelle le peroxyde d'hydrogène est présent en une quantité de 0,05 % à 0,2 % en poids de la composition de soin buccal ; et le peroxydisulfate est présent en une quantité de 5 % à 7 % en poids de la composition de soin buccal.

2. Composition de soin buccal selon la revendication 1, dans laquelle la composition est un dentifrice.

3. Composition de soin buccal selon la revendication 1 ou la revendication 2, dans laquelle le peroxyde d'hydrogène est présent en une quantité de 0,1 % en poids de la composition de soin buccal ; et dans laquelle le peroxydisulfate est présent en une quantité de 7 % en poids de la composition de soin buccal.

4. Composition de soin buccal selon l'une quelconque des revendications précédentes, dans laquelle le peroxydisulfate est le peroxydisulfate de sodium.

5. Composition de soin buccal selon l'une quelconque des revendications précédentes, dans laquelle la composition comprend :

un ou plusieurs humectants ;
un ou plusieurs épaississants ;
un ou plusieurs abrasifs ;
une ou plusieurs sources de fluorure ; et
un ou plusieurs détergents ou tensioactifs.

6. Composition de soin buccal selon l'une quelconque des revendications précédentes, dans laquelle la composition comprend :

du polyéthylène glycol en une quantité de 0 % à 23 % ;
de la glycérine (99,0 % à 101,0 %) en une quantité de 0 % à 32 % ;
du propylène glycol en une quantité de 1 % à 40 % ;
un copolymère de polyéthylèneglycol/polypropylèneglycol 116/66 en une quantité de 1 % à 10 % ;
de la polyvinylpyrrolidone en une quantité de 0,5 % à 10 % ;
du pyrophosphate de calcium en une quantité de 5 % à 30 % ;
de la silice, par exemple de la silice fumée, en une quantité de 0,1 % à 5 % ;
du laurylsulfate de sodium en une quantité de 1 % à 3 % ; et
du pyrophosphate tétrasodique en une quantité de 0 % à 3 %, en poids de la composition.

7. Composition de soin buccal selon la revendication 6, comprenant en outre de l'hydroxytoluène butylé en une quantité de 0,01 % à 0,05 % en poids de la composition.

8. Composition de soin buccal selon l'une quelconque des revendications précédentes, dans laquelle la composition comprend du peroxyde d'hydrogène en une quantité de 0,05 % à 0,15 % en poids de la composition.

9. Composition de soin buccal selon l'une quelconque des revendications précédentes, comprenant en outre un ou plusieurs adjuvants choisis parmi les agents édulcorants, les agents aromatisants et les agents colorants.

10. Composition de soin buccal selon l'une quelconque des revendications précédentes, pour une utilisation dans un procédé de blanchiment des dents, ledit procédé comprenant la mise en contact des dents avec la composition orale.

11. Utilisation d'un système de blanchiment selon la revendication 1 dans la fabrication d'une composition de soin buccal selon l'une quelconque des revendications 1 à 9.